# DEMANDE DE BREVET EUROPEEN

(11) **EP 2 080 481 A1**
(43) Date de publication de la demande: **22.07.2009**
(21) Numéro de dépôt: 09305047.4
(22) Date de dépôt: 20.01.2009
(51) Int. Cl.: A61B 17/435, A61B 19/00, A61K 49/22, A61L 29/14, A61M 25/00

(54) **Dispositif pour prélèvement ou transfert dans les organes génitaux présentant une visibilité aux ultra-sons améliorés**

(30) Priorité: 21.01.2008 FR 0800299
(71) Demandeur: Prodimed SAS, 60530 Neuilly-en-Thelle (FR)
(72) Inventeur: Bouveret, Patrick, F-60530, NEUILLY-EN-THELLE (FR); Berjat, Vincent, F-60820, BORAN-SUR-OISE (FR)
(74) Mandataire: Mouget-Goniot, Claire

(57) **Abrégé**

Dispositif dont la partie principale est un cathéter ou sonde qui présente l'avantage d'être constituée par un tube échogène sur sa totalité et proportionnellement transparent. La structure est constituée par un matériau choisi dans le groupe comprenant les matrices polymères thermoplastiques amorphes dans lequel est incorporée une tresse ou brin ou bande avec un matériau échogène non métallique, biocompatible et sélectionné parmi les polymères thermoplastique semi-cristallins chargés ou non chargés.

## Description

La présente invention concerne un dispositif médical échogène apte à permettre d'effectuer des prélèvements dans les organes génitaux, tels que des prélèvements de liquides physiologiques ou de fragments de la paroi interne d'organes génitaux, ou apte à permettre le transfert de produits, notamment de gamètes ou d'embryons, dans les organes génitaux femelles, notamment dans l'utérus ou les trompes.

Les dispositifs de prélèvement couramment utilisés actuellement, comprennent généralement un tube cylindrique en polymère(s) ouvert en ses deux extrémités, d'un diamètre extérieur d'environ 1 à 3 millimètres et de diamètre interne d'environ 0,5 à 2,8 millimètres, pour une longueur de 15 à 30 centimètres.

Ce tube cylindrique se présente de telle façon :
- qu'il peut être relié par le biais d'un raccord (tel qu'un cône Lüer ou Lüer Lock) à une seringue, ou à tout autre dispositif permettant de créer une dépression, une surpression, de recueillir ou d'injecter un produit ou un tissu.

Les dispositifs de transfert de gamètes ou de transfert d'embryons couramment utilisés actuellement sont des sondes intra-utérines comprenant généralement une sonde en polymère(s) transparent et souple de longueur d'environ 15 à 20 cm terminée par un cathéter très flexible à bout mousse et présentant deux ouvertures latérales opposées, ou une seule ouverture distale ; un raccord normalisé (tel qu'un cône Lüer ou Lüer Lock) permet la jonction de ce dispositif sur une seringue.

Ce type de dispositif permet d'effectuer une insémination artificielle intra utérine avec sperme préparé. La sonde facilite l'accès dans la cavité utérine sans traumatisme et permet de libérer les spermatozoïdes à proximité des ostiums tubaires.

En variante, des dispositifs de transfert d'embryons comprennent :
- un cathéter d'introduction, en polymère(s), ayant environ 15 à 25 cm de long, un diamètre externe d'environ 2 à 3 mm et étant avantageusement muni d'une bague coulissante (butée),
- un cathéter de réimplantation, en polymère, ayant environ 15 à 30 cm présentant des graduations repères espacées d'un centimètre à sa partie inférieure.

L'utilisation, en général à usage unique, de ce dispositif de transfert connu est la suivante : on fait pénétrer le cathéter introducteur jusqu'à l'orifice interne du col de l'utérus.

Simultanément, le ou les embryons ont été chargés par le biologiste dans le cathéter de réimplantation.

Ce cathéter de réimplantation est alors introduit dans l'introducteur et sera poussé jusqu'au lieu de transfert.

Lorsque la première marque déterminée du cathéter de réimplantation affleure l'extrémité proximale du cône Lüer de l'introducteur, leurs deux extrémités distales coïncident. Les marques portées sur l'introducteur à partir de son extrémité distale permettent de calculer la longueur introduite dans l'utérus. Il faudra ajouter le dépassement du cathéter de réimplantation (nombre de cm dont on a dépassé les marques de concordance).

Les dispositifs de prélèvement ou de transfert tels que définis ci-dessus sont difficiles à localiser dans la cavité utérine, la précision de l'endroit où seront déposés les embryons ou les gamètes conditionnant les taux de réussite des transferts ainsi que la précision des prélèvements et plusieurs solutions ont été proposées pour améliorer l'échogénicité de ses dispositifs, c'est-à-dire leur capacité à être identifiables sous ultrasons.

Ainsi la demande GB 2 379 610 décrit l'introduction de bulles de gaz dans toute l'épaisseur du matériau plastique utilisé dans des dispositifs médicaux. La demande internationale WO 00/09178 décrit un dispositif médical dont une partie est rendue échogène par incorporation de microparticules métalliques d'une taille inférieure à 500 nm et la demande EP 1 462 056 divulgue un cathéter dont la gaine comprend une couche externe contenant des microsphères de gaz isopentane et une couche interne sans bulles. La demande internationale WO 2005/035043 décrit un dispositif à mémoire de forme utilisé comme dispositif vaso-occlusif dont la partie principale est un tube comprenant une structure constituée par un matériau principalement magnétique et modérément échogène. Cette partie peut être constituée par une matrice polymère thermoplastique chargé avec des particules métalliques (tungstène, palladium, or, argent, oxyde de fer etc..) ou bien dans laquelle sont piégées des bulles. La zone ainsi réalisée est opaque.

Néanmoins l'échogénicité de ces différents dispositifs n'est pas optimale et il y a nécessité de disposer de dispositifs présentant des performances améliorées.

La présente invention a pour but de fournir des dispositifs de prélèvement ou de transfert présentant l'avantage, par rapport aux dispositifs existants décrits ci-dessus, de posséder un caractère échogène plus marqué sur toute sa longueur et également au niveau de l'orifice utile de ces dispositifs pour le prélèvement ou le transfert. La partie principale qui compose le cathéter est élaborée à partir d'un tube transparent constitué d'une matrice polymère thermoplastique imprégnée ou formulée au moyen d'un matériau susceptible de générer des discontinuités acoustiques. Cette amélioration permet la manipulation du dispositif avec plus d'aisance et de finesse lors de l'acte médical (positionnement bien plus précis, que ne le permettaient les dispositifs décrits ci-dessus, de la zone où se fera le prélèvement ou le transfert). D'autre part cela permet au praticien ne plus travailler en aveugle comme cela est le cas actuellement. L'amélioration de la visualisation permet de positionner des embryons avec précision dans la cavité utérine contribuant ainsi à améliorer le taux de réussite des transferts.

L'invention a également pour but de fournir des dispositifs de prélèvement ou de transfert présentant l'avantage, par rapport aux dispositifs existants décrits ci-dessus, d'être tels que le matériau échogène n'est jamais en contact avec les produits prélevés ou transférés, afin de ne pas altérer leurs caractéristiques biochimiques ni leur état physiologique.

L'invention concerne un dispositif dont la partie principale est un cathéter élaboré à partir d'un tube échogène sur sa totalité et proportionnellement transparent, ledit matériau échogène étant choisi dans le groupe comprenant les matrices polymères thermoplastiques amorphes dans lesquelles est incorporée une tresse ou un brin ou une bande en matériau échogène non métallique et biocompatible.

On entend par proportionnellement transparent le fait que le tube ne soit pas complètement opaque mais que des zones transparentes sont aussi présentes, la discontinuité acoustique étant lié à la différence de masse volumique (compacité) des deux matériaux (différence d'impédance), à savoir la matrice polymère et le matériau constituant la tresse, le brin ou la bande. Les zones transparentes ainsi présentes permettent au biologiste de vérifier, par exemple, la présence des embryons dans le cathéter avant le transfert et de constater également l'absence de ces embryons après réimplantation témoignant ainsi de l'accomplissement du transfert.

Parmi les matrices polymères amorphes, on peut citer notamment les polyuréthanes aliphatiques base polyéther. Ces matrices sont avantageusement utilisées lorsque le dispositif est destiné à une application médicale.

La tresse ou brin ou bande imprégnée est constituée par un matériau non métallique, biocompatible et avantageusement sélectionné parmi les polymères thermoplastiques semi-cristallins (polyamides, polyesters par exemple) ou parmi les matériaux thermoplastiques chargés (sulfate de baryum, composé à base de borosilicate par exemple).

Dans un mode particulier de l'invention, la tresse est constituée par un entrelacement jointif de brins afin d'obtenir une géométrie régulière et adaptable (maillage). La densité de maille est maîtrisée et ajustable au besoin (plus ou moins large) afin de permettre la visualisation au travers du tube. Le tube ainsi constitué est échogène sur toute la longueur et non opaque du fait de la présence de mailles et de zones régulières et transparentes.

La tresse imprégnée est en général constituée d'un monofilament ou brin dont le diamètre est compris avantageusement entre 0,05 et 0,10 mm de diamètre.

En outre la topographie de la surface interne du tube constituant le cathéter est parfaitement lisse et prévient toute adhérence sur la surface interne. Ceci rend les dispositifs, définis selon l'invention, particulièrement adaptés au transfert de gamètes, et d'embryons ou au prélèvement de fragment de parois. La qualité de l'imagerie échographique est significativement améliorée compte tenu de l'augmentation des échos et de la nature différente des discontinuités acoustiques générées.

Les dispositifs selon l'invention sont aptes à permettre d'effectuer des prélèvements dans les organes génitaux en vue d'analyse, tels que des prélèvements de liquides physiologiques ou de fragments de la paroi interne d'organes génitaux mâles ou femelles, plus particulièrement d'organes génitaux féminins, notamment au niveau du col de l'utérus, de l'utérus ou des trompes, ou apte à permettre le transfert de produits tels que ceux choisis parmi les gamètes, ou les embryons, ou les principes actifs, ou les produits utilisés en radiologie dans les organes génitaux femelles, notamment dans les organes génitaux féminins susmentionnés.

Les dispositifs selon l'invention sont constitués d'un cathéter monovoie (canal unique) ouvert dans sa partie distale au niveau de l'orifice utile, à l'intérieur duquel circulent le liquide et l'embryon.

Par orifice utile, on entend conformément à l'invention le ou les trous qui, dans les dispositifs susmentionnés, sont ceux par lesquels les prélèvements sont aspirés, ou les gamètes, embryons, principes actifs, ou produits de contraste sont expulsés.

Dans un mode de réalisation avantageux de l'invention, le dispositif comprend :
- le cathéter correspondant à un tube cylindrique dont l'extrémité distale est :
   - soit ouverte et constitue l'orifice utile pour le prélèvement de liquides physiologiques, de fragments de cellules, de tissus ou de parois d'organes génitaux, ou le transfert de produits tels que les gamètes, embryons, principes actifs, ou produits de contraste,
   - soit obturée à l'exception d'au moins un trou dit d'aspiration, le cas échéant situé en position latérale de l'extrémité distale dudit cathéter, et constituant l'orifice utile pour le prélèvement liquides physiologiques, de fragments de cellules, de tissus ou de parois d'organes génitaux, ou le transfert de produits tels que les gamètes, embryons, principes actifs, ou produits de contraste,
- un piston étanche apte à se déplacer dans ledit tube cylindrique, et relié à l'extrémité distale d'une tige dont l'extrémité proximale est avantageusement pourvue d'un organe de préhension, ou d'une seringue ou d'un autre système de vide permettant de créer une dépression ou pression dans le tube échogène.

La longueur des cathéters de prélèvement, de transfert, ou d'essais susmentionnés est de préférence comprise entre environ 150 à environ 300 mm.

L'invention a également pour objet l'utilisation d'un dispositif tel que décrit précédemment pour permettre d'effectuer des prélèvements dans les organes génitaux en vue d'analyse ou apte à permettre le transfert de produits, notamment d'embryons ou de gamètes.

Les dispositifs selon l'invention peuvent être réalisés selon toute technique connue de l'homme du métier. Ainsi le procédé de préparation peut comprendre les étapes suivantes :
- fabrication de la tresse dans le cas où on utilise une tresse,
- fabrication des tubes échogènes par extrusion et imprégnation dans le cas des structures avec tresse ou par co-extrusion dans le cas des structures avec une bande ou un brin échogène,
- effilage atraumatique de l'extrémité des tubes ainsi obtenus.

Dans un mode de réalisation avantageux du procédé de l'invention, la fabrication de la tresse et l'imprégnation dans le tube se font simultanément en une seule étape par un procédé d'extrusion des polymères thermoplastiques. Il est également possible, si on le souhaite, de générer des microcavités au cours de cette étape.

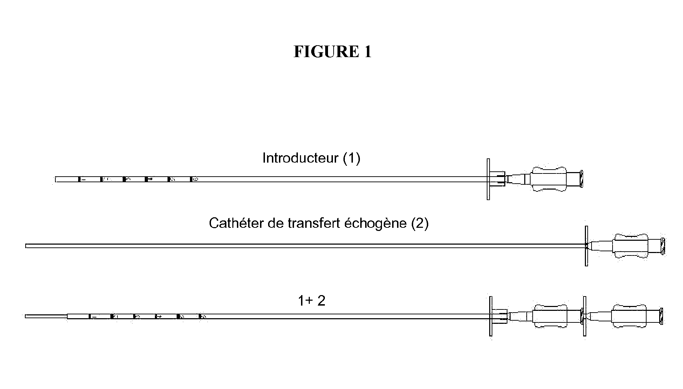

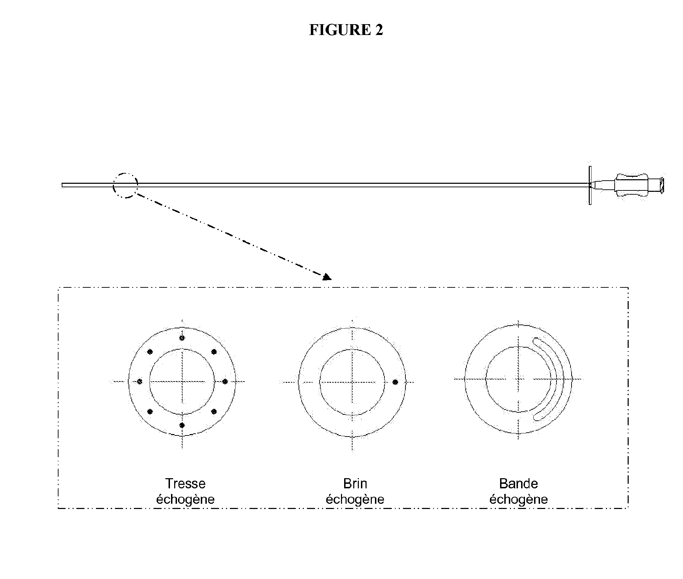

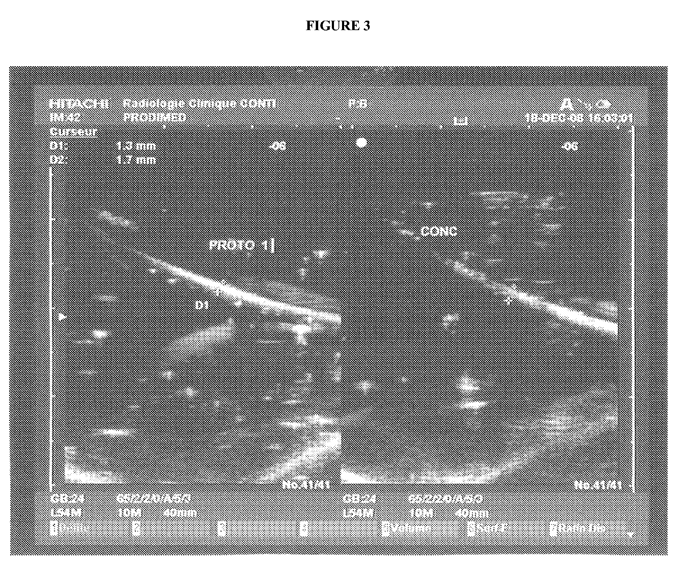

L'invention sera davantage détaillée à l'aide des figures 1 à 3.
La figure 1 montre un exemple de dispositif pour le transfert d'embryon selon l'invention. L'introducteur (1) porte des marques de positionnement non échogènes.
La figure 2 montre la coupe transversale du cathéter de transfert échogène via la présence d'une tresse, d'une bande ou d'un brin à caractère échogène.
La figure 3 est une échographie comparative entre le dispositif selon l'invention (PROTO 1) et un dispositif de l'état antérieur de la technique (CONC).

Les dispositifs selon l'invention possèdent un caractère échogène plus marqué sur toute leur longueur, beaucoup plus homogène et notamment au niveau de l'orifice utile pour le prélèvement ou le transfert.

## Revendications

1. Dispositif, notamment pour transfert d'embryons, dont la partie principale est le cathéter élaboré à partir d'un tube échogène sur sa totalité et proportionnellement transparent, ledit matériau échogène étant choisi dans le groupe des matériaux comprenant des matrices polymères thermoplastiques amorphes dans lesquelles est incorporée une tresse ou un brin ou une bande en matériau échogène non métallique et biocompatible.

2. Dispositif échogène selon la revendication 1 **caractérisé en ce que** la tresse, le brin ou la bande est constituée par un matériau non métallique choisi dans le groupe comprenant les polymères thermoplastique semi-cristallins et les matériaux thermoplastiques chargés.

3. Dispositif selon la revendication 2 **caractérisé en ce que** le polymère thermoplastique semi-cristallin est choisi parmi les polyamides et les polyesters

4. Dispositif selon la revendication 2 **caractérisé en ce que** les matériaux thermoplastiques chargés le sont avec des particules sélectionnées parmi le sulfate de baryum et les composés à base de borosilicate.

5. Dispositif échogène selon la revendication 1 **caractérisé en ce que** le cathéter de transfert ou de prélèvement est un tube constitué par une matrice polymère thermoplastique amorphe à base de polyuréthane aliphatique base polyéther dans laquelle est incorporée une tresse, ou brin, ou bande échogène non métallique, ledit dispositif étant adapté spécifiquement pour les applications médicales.

6. Dispositif échogène selon la revendication 1 **caractérisé en ce que** la tresse imprégné est constituée d'un monofilament ou brin dont le diamètre est compris entre 0,01 et 0,10 mm de diamètre, avantageusement entre 0,05 et 0,10 mm.

7. Dispositif selon la revendication 1 **caractérisé en ce qu'**il comprend :
- le cathéter qui est un tube cylindrique dont l'extrémité distale est :
• soit ouverte et constitue l'orifice utile pour le prélèvement de liquides physiologiques, de fragments de cellules, de tissus ou de parois d'organes génitaux, ou le transfert de produits tels que les gamètes, embryons, principes actifs, ou produits radiologiques,
• soit obturée à l'exception d'au moins un trou dit d'aspiration, le cas échéant situé en position latérale de l'extrémité distale dudit cathéter, et constituant l'orifice utile pour le prélèvement liquides physiologiques, de fragments de cellules, de tissus ou de parois d'organes génitaux, ou le transfert de produits tels que les gamètes, embryons, principes actifs, ou produits de contraste,
- un piston étanche apte à se déplacer dans ledit tube cylindrique, et relié à l'extrémité distale d'une tige dont l'extrémité proximale est avantageusement pourvue d'un organe de préhension, ou d'une seringue ou d'un autre système de vide permettant de créer une dépression ou pression dans le tube échogène.
